Europäisches Patentamt

(19) European Patent Office

Office européen des brevets

(11) EP 0 539 432 B1

(12) **EUROPEAN PATENT SPECIFICATION**

(45) Date of publication and mention
of the grant of the patent:
**25.11.1998 Bulletin 1998/48**

(21) Application number: **91912962.7**

(22) Date of filing: **18.07.1991**

(51) Int. Cl.[6]: **A61K 49/00**

(86) International application number:
**PCT/GB91/01190**

(87) International publication number:
**WO 92/01478 (06.02.1992 Gazette 1992/04)**

(54) **DIAGNOSTIC COMPOSITIONS FOR ASSESSMENT OF PANCREATIC INSUFFICIENCY**

DIAGNOSTISCHES MITTEL ZUR BESTIMMUNG VON PANKREASINSUFFIZIENS

COMPOSES SERVANT AU DIAGNOSTIC D'EVALUATION DE L'INSUFFISANCE PANCREATIQUE

(84) Designated Contracting States:
**AT BE CH DE DK ES FR GR IT LI LU NL SE**

(30) Priority: **19.07.1990 GB 9015851**
**27.06.1991 GB 9114042**

(43) Date of publication of application:
**05.05.1993 Bulletin 1993/18**

(73) Proprietor: **UNICLIFFE LIMITED**
**Stonar, Kent CT13 9NJ (GB)**

(72) Inventors:
• **JOLLIFEE, Stephen R.,**
**Charwell Pharm. Lim.**
**Hampshire GU3 2TJ (GB)**

• **WEAVER, Lawrence T.,**
**Dunn Nutritional Lab.**
**Cambridge CB4 1XJ (GB)**

(74) Representative:
**Woodcraft, David Charles et al**
**High Holborn House**
**52/54 High Holborn**
**London WC1V 6SE (GB)**

(56) References cited:
EP-A- 312 108          WO-A-89/05638
DE-B- 1 598 833        US-A- 3 806 592
US-A- 3 917 812

• **The Lancet, Vol. 2, October 1982 Ralph E. Barry**
**et al.: "Fluorescein dilaurate- tubeless test for**
**pancreatic exocrine failure "**

## Description

This invention relates to diagnostic compositions for detecting pancreatic insufficiency.

Identification of pancreatic insufficiency is a useful tool in diagnosis of a variety of diseases, including pancreatitis and cystic fibrosis. One test which has been widely used for routine detection of pancreatic insufficiency involves the administration of fluorescein dilaurate (herein referred to as FDL) When this compound comes into contact with pancreatic juices it is broken down by pancreas-specific cholesterol ester hydrolase into fluorescein and two molecules of lauric acid. The coloured, water-soluble fluorescein is absorbed and excreted in the urine. If the patient's urine is collected over a ten hour period, and the fluorescein content determined, this provides an indirect measure of glandular output of the exocrine pancreas. This procedure is described, for example in Barry et al, The Lancet 1982, Vol. 2, pages 742 to 744.

One disadvantage which is inherent in the above-described pancreolauryl test is that it requires to be carried out over a two day period, since the amount of fluorescein which is excreted must be compared with the amount excreted when an equivalent amount of non-esterified fluorescein is administered to the patient. This control test enables the physician to compensate for any individual absorption or excretion characteristics which could interfere with the result.

Thus, while effective and suitable for routine diagnostic testing, the pancreolauryl test does have two disadvantages, namely, that it is necessary to carry out a control test and secondly the whole of the urine excreted by the patient after administration of the diagnostic composition must be carefully collected.

The present invention is based upon the discovery that by incorporating a particular type of permeability marker in the diagnostic composition, it becomes no longer necessary to carry out a control test, since determination of the marker simultaneously with determination of the fluorescein enables the technician or physician to compensate for differences in absorption of the active materials and excretion of the fluorescein and permeability marker by individual patients.

According to the present invention there is provided a diagnostic composition for detecting pancreatic insufficiency which comprises a fatty acid diester of fluorescein and a saccharide selected from mannitol, lactulose, xylose, rhamnose and fructose.

Preferably, the di-fatty acid ester is fluorescein dilaurate (hereinafter referred to as FDL), since this is readily broken down by the enzyme present in pancreatic juices produced by a healthy pancreas. The saccharide is mannitol, lactulose, rhamnose, xylose or fructose of which mannitol is most preferred. Mannitol and lactulose have the particular property that they are highly resistant to break down in the stomach and absorption through the intestinal mucosa seems to be substantially passive. In other words, the transport of the saccharide through the intestinal wall does not seem to involve any reaction with any other material in the digestive system.

Preferably, the saccharide, e.g. the mannitol, is present in the diagnostic composition in a molar excess compared with the FDL. Preferably, the molar ratio of FDL to mannitol is from 1:5 to 1:100, more preferably 1:10 to 1:60, e.g. 1:50.

Although various dosage forms of the diagnostic test kits may be used, e.g. encapsulated compositions or tabletted solid compositions, such dosage forms are usually unsuitable for children (including neonates) or indeed for adult patients who have swallowing difficulties. However, because the fluorescein fatty acid ester is water-insoluble, addition of the marker compound to a liquid feed, such as milk, or a watery drink is not possible.

In a preferred form of the invention as applied to liquid formulations, there is provided an emulsified diagnostic composition for detecting pancreatic insufficiency which comprises an oil phase dispersed in an aqueous phase, the oil phase having a di-fatty acid ester of fluorescein dissolved therein and the composition including a saccharide which is resistant to breakdown within the stomach and which does not take part in any active transport system across the intestinal mucosa.

Emulsions prepared in accordance with the invention may be readily added to an infant's drink and an accurate pancreatic insufficiency assessment carried out. In the case of emulsions which additionally contain a saccharide or other metabolic resistant second marker, no control test need, of course, be carried out.

The emulsions are prepared by dissolving the fluorescein di-fatty acid ester (preferably the dilaurate) in a suitable oil phase. Triglycerides have been found suitable, especially those of medium chain length. One specific example is a fractionated coconut oil obtainable under the trade name 'Miglyol 812' from Dynamit Nobel. These dissolve the fluorescein ester without presenting absorption problems to patients suffering from cystic fibrosis.

Having dissolved the fluorescein di-ester in the triglyceride, an oil-in-water emulsion is formed by dispersing the triglyceride as fine droplets in the aqueous phase. The saccharide may be pre-dissolved in the water. An emulsion is preferably formed by use of ultrasonic energy, although other dispersing methods may be used to obtain emulsion homogenisation. Ultrasonic dispersion has the advantage that an extremely fine dispersion of oil droplets is formed in the aqueous phase. This 'micro-emulsion' system enhances the physical stability of the emulsion, important in paediatric formulations, where the choice of acceptable emulsifiers is limited. Moreover, micro-emulsions require little effort to form an homogenous mixture when added to an infant drink. Suitable stabilizing surfactants include lecithin, e.g. soya bean lecithins which are obtainable from Lipoid K.G. and Lucas Meyer under the trade names 'Lipoid 505' and 'Topcithin', respectively.

The emulsions are prepared in dosage unit forms so that a single dosage unit can be added to a standard infant feed, e.g. a 150 ml feed.

The fluorescein di-fatty acid ester may be present in an amount of from about 0.1 to 0.5 m.moles, preferably about 0.125 m.moles.

The saccharide is, of course, present to provide a means of measuring gastro-intestinal permeability. The saccharide should be non-metabolisable and the preferred saccharide is mannitol. Alternatives include lactulose, xylose and rhamnose. The saccharide should be present in molar excess compared with the fluorescein ester. Typically, the saccharide is present in the emulsion in concentrations of from 1 to 10 m.moles, e.g. about 6.25 m.moles. The resulting emulsions are filled into boro-silicate vials in the desired unit doses, sealed and autoclaved to sterilise them.

The following specific formulations of emulsion formulations will illustrate the invention. In all cases, the FDL was pre-dissolved in the Miglyol 812, the remaining components were added and emulsified using an ultrasonic disperser.

Example 1

| Dual Marker System | | |
|---|---|---|
| Component | mg/dose | % w/w |
| Fluorescein Dilaurate | 87.00 | 0.87 |
| Mannitol B.P. | 1138.00 | 11.38 |
| Miglyol 812 | 1250.00 | 12.50 |
| Lipoid S05 | 250.00 | 2.50 |
| Water (mains, boiled) | 7275.00 | 72.65 |
| | 10000.00 | 100.00 |

As stated above, the diagnostic composition may be administered as an encapsulated solid composition or a tabletted composition which may be administered in one or more tablets or capsules. Typically, a dosage unit of the FDL is from 0.2 to 0.8 mmols and the mannitol from about 20 to 30 mmols with FDL and mannitol being typically in the molar ratio of 1:50. The FDL and Mannitol may be intimately mixed to form a homogeneous powder and filled into hard gelatin capsules. Suitable flow and compaction aids, commonly known to those familiar to capsule technology, may be added to assist production.

An example of an encapsulated formulation is as follows:

| Component | mg/dose | % w/w |
|---|---|---|
| Fluorescein Dilaurate | 87.00 | 7.03 |
| Mannitol | 1138.00 | 91.97 |
| Aerosil 200 (colloidal silicon dioxide) | 12.25 | 1.00 |
| | 1237.25 | 100.00 |

The powder mix may be filled into two/three size 00 hard gelatin capsules.

The formulation illustrated above is intended for infants and is one-quarter of the standard adult does. A standard adult dose would therefore be 0.5m Moles (348.5 mg) of FDL and 25m Moles (4552.0 mg) of Mannitol contained in the appropriate number of capsules.

It has been found that after administration of the diagnostic composition a steady state is reached after about 4 to 6 hours from administration, at which time a sample may be taken and analysed for fluorescein and mannitol contents. The ratio of fluorescein to mannitol in the sample gives a clear discrimination between normal pancreatic activity and pancreatic insufficiency.

Fluorescein may be conveniently analyzed quantitatively in a sample using a spectrophotometer. The evaluation can be carried out directly by measuring the absorption of a sample of urine containing the excreted fluorescein and mannitol. In the case of mannitol determination, this is conveniently carried out by the method described in the paper by Lunn et al, published in Clinica Chimica Acta, 1989, CCA04504. In this method, mannitol is treated with mannitol dehydrogenase which is extracted from a culture of a bacterium NCIB NCTC 6992, obtainable from NCIB Torry Research Station, Aberdeen Scotland. This particular enzyme converts mannitol into fructose and NADH which can be determined by optical density measurements at 340 nm. The relationship between the original mannitol concentration and the optical density of a sample treated with the bacterium is almost exactly linear, so that the optical density can be calibrated in mmols of mannitol Thus, samples of the excreted urine after administration of the diagnostic composition can be determined for fluorescein and mannitol concentrations by a simple spectrophotometric analysis before and after addition of the enzyme which converts the mannitol into fructose.

In order to demonstrate the operation of the diagnostic method, the following study was carried out of 6 healthy adults (four male and two female) aged 29 to 40 years which constituted group 1. Six patients diagnosed as having cystic fibrosis (five male and one female aged 7 to 21 years) were tested both before having been supplied with pancreatic enzyme supplement (group 2) and after having been supplied with such a supplement (group 3).

After an overnight fast, each of the patients ingested an encapsulated composition comprising 0.5 mmols of FDL and 25 mmols of mannitol at time zero followed by a standardized breakfast consisting of 50 g of bread spread with 20 g of butter and 1 cup of liquid.

Urine was collected for ten hours in 2 hourly aliquots and urinary fluorescein was measured photometrically and mannitol was measured enzymatically and photometrically as described above. There was no significant difference in mean (standard deviation) ten hour urine volume which were 1475 (278 mmls) group 1, 1008 (398 mmls) group 2 and 849 (536 mmls) for group 3. There was also no significant difference in percentage mannitol recovery 35.0 (11.1%) group 1, 40.1 (18.1%) group and 28.6 (7.0%) group 3. However, there was a pronounced difference in percent fluorescein recoveries between the three groups, i.e. 18.7 (3.8%) group 1, 1.2 (0.5 %) group 2 and 0.8 (0.3%) group 3, resulting in a significant difference in ten hourly fluorescein to mannitol ratios 57.3 (18.2). 3.4 (1.4), 3.1 (1.9) respectively.

These results are shown schematically in the attached bar charts. When the results were analysed in two hour urine aliquots it was seen that there was a delay in urinary recovery of fluorescein versus mannitol which is due to the pancreatic digestive phase in both controls and cystic fibrosis groups. Differences in urinary fluorescein to mannitol ratios between controls and cystic fibrosis patients (both those with and without pancreatic supplements) achieved biological and statistical significance from 2 to 8 hours post ingestion of the diagnostic composition. These results show that the addition of mannitol to FDL permits clear distinction between subjects with normal pancreatic function and those with cystic fibrosis whether receiving pancreatic enzyme supplements or not. Thus, by using the new diagnostic test it is possible to detect pancreatic insufficiency by taking samples between 2 and 8 hours after ingestion of the composition without the need to run a further control.

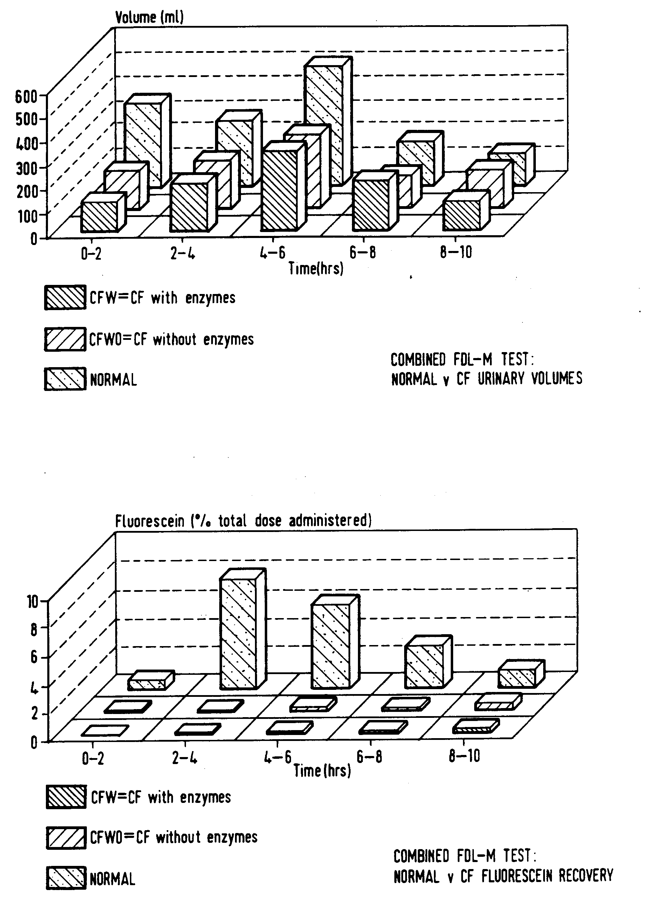

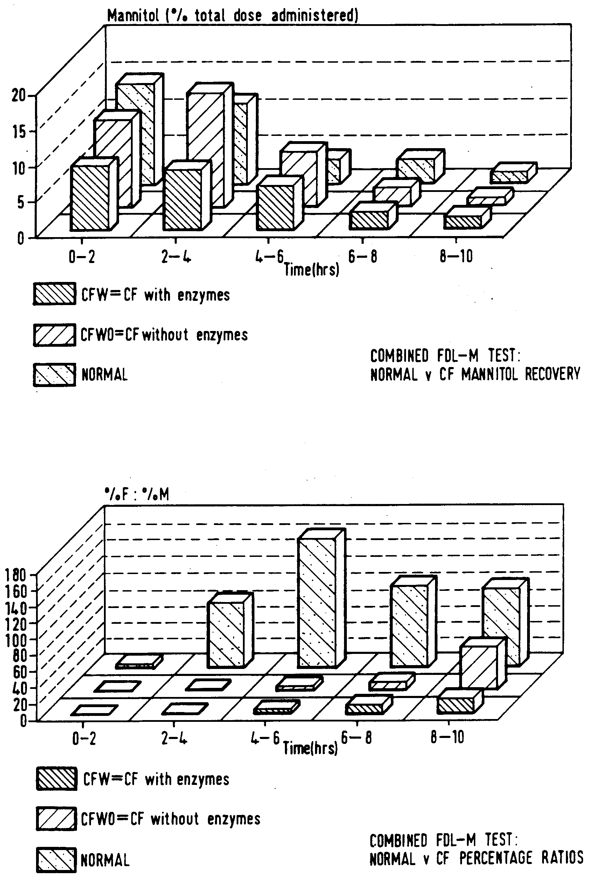

## Claims

### Claims for the following Contracting States : AT, BE, CH, DE, DK, FR, GB, IT, LI, LU, NL, SE

1. A diagnostic composition for detecting pancreatic insufficiency which comprises a fatty acid diester of fluorescein and a saccharide selected from mannitol, lactulose, xylose, rhamnose and fructose.

2. A composition according to claim 1 wherein the fatty acid ester is fluorescein dilaurate.

3. A composition according to claim 1 or claim 2 wherein the fluorescein ester and the saccharide are present in a molar proportion of about 1:5 to about 1:100.

4. A composition according to any one of the preceding claims which is in the form of an emulsion.

5. A composition according to claim 4 wherein the emulsion comprises an oil phase dispersed in a continuous aqueous phase containing the saccharide, the oil phase having dissolved therein a di-fatty acid ester of fluorescein.

6. A composition according to claim 5 in which the fluorescein ester and the saccharide are present in a molar proportion of about 1:5 to about 1:100.

7. A composition according to claim 5 in which the fluorescein ester and the saccharide are present in a molar proportion of from about 1:10 to about 1:60.

8. A composition according to any one of claims 5 to 7 which is in the form of a concentrate intended for addition to a liquid feed and wherein the fluorescein ester is present in the emulsion in a concentration of from about 0:1 to 0:5 m.moles.

9. A composition according to any one of claims 5 to 8 in which the saccharide is present in a concentration of from about 1 to 10 m.moles.

10. A composition according to any one of claims 5 to 9 in which the fluorescein ester is dissolved in a triglyceride.

11. Use of a fatty acid diester of fluorescein and a saccharide selected from mannitol, lactulose, xylose, rhamnose and fructose in the manufacture of a diagnostic agent for detecting pancreatic insufficiency.

**Claims for the following Contracting States : ES, GR**

1. A method of producing a diagnostic composition for detecting pancreatic insufficiency comprising the step of mixing

    i. a fatty acid diester of fluorescein with;
    ii. a saccharide selected from mannitol, lactulose, xylose, rhamnose and fructose.

2. A method as claimed in claim 1 wherein the fatty acid diester is fluorescein dilaurate.

3. A method as claimed in claim 1 or claim 2 where the molar proportion of the fluorescein ester and saccharide is about 1:5 to about 1:100.

4. A method as claimed in any one of the preceding claims comprising the further step of forming an emulsion comprising an oil phase having dissolved therein the fatty acid diester dispersed in a continuous aqueous phase containing the saccharide.

5. A method as claimed in claim 4 wherein the molar proportion of fluorescein ester to saccharide is from about 1:10 to about 1:60.

6. A method as claimed in any one of the preceding claims wherein the fluorescein ester is dissolved in a triglyceride.

7. Use of a fatty acid diester of fluorescein and a saccharide selected from mannitol, lactulose, xylose, rhamnose and fructose to manufacture a diagnostic agent for detection of pancreatic insufficiency.

**Patentansprüche**

**Patentansprüche für folgende Vertragsstaaten : AT, BE, CH, DE, DK, FR, GB, IT, LI, LU, NL, SE**

1. Diagnoseverbindung zur Feststellung einer Insuffizienz der Bauchspeicheldrüse, die ein Fettsäurediester des Fluoresceins und ein Saccharid umfaßt, das aus der aus Mannitol, Lactulose, Xylose, Rhamnose und Fructose bestehenden Gruppe gewählt wird.

2. Verbindung nach Anspruch 1, wobei der Fettsäureester Fluoresceindilaurat ist.

3. Verbindung nach Anspruch 1 oder 2, wobei der Fluoresceinester und das Saccharid in einem Molverhältnis von etwa 1:5 bis etwa 1:100 vorliegen.

4. Verbindung nach einem der vorhergehenden Ansprüche, die in Form einer Emulsion vorliegt.

5. Verbindung nach Anspruch 4, wobei die Emulsion eine Ölphase umfaßt, die in einer das Saccharid enthaltenden zusammenhängenden wässrigen Phase dispergiert ist und den gelösten Di-Fettsäureester des Fluoresceins umfaßt.

6. Verbindung nach Anspruch 5, wobei der Fluoresceinester und das Saccharid in einem Molverhältnis von etwa 1:5 bis etwa 1:100 vorliegen.

7. Verbindung nach Anspruch 5, wobei der Fluoresceinester und das Saccharid in einem Molverhältnis von etwa 1:10 bis etwa 1:60 vorliegen.

8. Verbindung nach einem der Ansprüche 5 bis 7, die in Form eines Konzentrates vorliegt, das einem flüssigen Nahrungsmittel oder einem flüssigen Ansatz hinzugefügt wird, wobei die Konzentration des Fluoresceinesters in der Emulsion etwa 0,1 bis 0,5 Millimol beträgt.

9. Verbindung nach einem der Ansprüche 5 bis 8, wobei die Konzentration des Saccharids etwa 1 bis 10 Millimol beträgt.

10. Verbindung nach einem der Ansprüche 5 bis 9, wobei das Fluoresceinester in Triglycerid gelöst ist.

11. Verwendung eines Fettsäurediesters des Fluoresceins und eines Saccharids, das aus der aus Mannitol, Lactulose, Xylose, Rhamnose und Fructose bestehenden Gruppe ausgewählt wird, zur Herstellung eines Diagnosemittels zur Feststellung einer Insuffizienz der Bauchspeicheldrüse.

**Patentansprüche für folgende Vertragsstaaten : ES, GR**

1. Verfahren zur Herstellung einer Diagnoseverbindung zur Feststellung einer Insuffizienz der Bauchspeicheldrüse durch Mischen eines Fettsäurediesters des Fluoresceins mit einem Saccharid, das aus der aus Mannitol, Lactulose, Xylose, Rhamnose und Fructose bestehenden Gruppe ausgewählt wird.

2. Verfahren nach Anspruch 1, wobei der Fettsäurediester Fluoresceindilaurat ist.

3. Verfahren nach Anspruch 1 oder 2, wobei der Fluoresceinester und das Saccharid in einem Molverhältnis von etwa 1:5 bis etwa 1:100 vorliegen.

4. Verfahren nach einem der vorhergehenden Ansprüche mit dem zusätzlichen Verfahrensschritt der Bildung einer Emulsion, die eine Ölphase umfaßt, in der der Fettsäurediester gelöst ist, der in einer das Saccharid enthaltenden zusammenhängenden wässrigen Phase dispergiert ist.

5. Verfahren nach Anspruch 4, wobei der Fluoresceinester und das Saccharid in einem Molverhältnis von etwa 1:10 bis 1:60 vorliegen.

6. Verfahren nach einem der vorhergehenden Ansprüche, wobei der Fluoresceinester in Triglycerid gelöst ist.

7. Verwendung eines Fettsäurediesters des Fluoresceins und eines Saccharids, das aus der aus Mannitol, Lactulose, Xylose, Rhamnose und Fructose bestehenden Gruppe ausgewählt wird, zur Herstellung eines Diagnosemittels zur Feststellung einer Insuffizienz der Bauchspeicheldrüse.

**Revendications**

**Revendications pour les Etats contractants suivants : AT, BE, CH, DE, DK, FR, GB, IT, LI, LU, NL, SE**

1. Composition diagnostique pour détecter une insuffisance pancréatique, qui comprend un diester d'acide gras de fluorescéine et un saccharide choisi parmi le mannitol, le lactulose, le xylose, le rhamnose et le fructose.

2. Composition selon la revendication 1, dans lequel l'ester d'acide gras est le dilaurate de fluorescéine.

3. Composition selon la revendication 1 ou la revendication 2, dans lequel l'ester de fluorescéine et le saccharide sont présents en une proportion molaire d'environ 1/5 à environ 1/100.

4. Composition selon l'une quelconque des revendications précédentes, qui est sous forme d'une émulsion.

5. Composition selon la revendication 4, dans laquelle l'émulsion comprend une phase huileuse dispersée dans une phase aqueuse continue contenant le saccharide, la phase huileuse ayant, dissous en son sein, un diester d'acide gras de fluorescéine.

6. Composition selon la revendication 5, dans laquelle l'ester de fluorescéine et le saccharide sont présents en une proportion molaire d'environ 1/5 à environ 1/100.

**7.** Composition selon la revendication 5, dans laquelle l'ester de fluorescéine et le saccharide sont présents en une proportion molaire d'environ 1/10 à environ 1/60.

**8.** Composition selon l'une quelconque des revendications 5 à 7, qui est sous forme d'un concentré destiné à être ajouté à un aliment liquide et dans laquelle l'ester de fluorescéine est présent dans l'émulsion à une concentration d'environ 0,1 à 0,5 m.moles.

**9.** Composition selon l'une quelconque des revendications 5 à 8 dans laquelle le saccharide est présent à une concentration d'environ 1 à 10 m.moles.

**10.** Composition selon l'une quelconque des revendication 5 à 9, dans laquelle l'ester de fluorescéine est dissous dans un triglycéride.

**11.** Utilisation d'un diester d'acide gras de fluorescéine et d'un saccharide choisi parmi le mannitol, le lactulose, le xylose, le rhamnose et le fructose dans la fabrication d'un agent diagnostique pour détecter une insuffisance pancréatique.

**Revendications pour les Etats contractants suivants : ES, GR**

**1.** Méthode de production d'une composition diagnostique pour détecter une insuffisance pancréatique comprenant l'étape consistant à mélanger

    i. un diester d'acide gras de fluorescéine avec
    ii. un saccharide choisi parmi le mannitol, le lactulose, le xylose, le rhamnose et le fructose.

**2.** Méthode selon la revendication 1, dans lequel l'ester d'acide gras est le dilaurate de fluorescéine.

**3.** Méthode selon la revendication 1 ou la revendication 2, dans laquelle la proportion molaire d'ester de fluorescéine et de saccharide est d'environ 1/5 à environ 1/100.

**4.** Méthode telle que revendiquée dans l'une quelconque des revendications précédentes, comprenant l'étape supplémentaire de formation d'une émulsion comprenant une phase huileuse ayant, dissous en son sein, un diester d'acide gras de fluorescéine, dispersée dans une phase aqueuse contenant le saccharide.

**5.** Méthode telle que revendiquée dans la revendication 4, dans laquelle la proportion molaire est d'environ 1/10 à environ 1/60.

**6.** Méthode telle que revendiquée dans l'une quelconque des revendications précédentes, dans laquelle l'ester de fluorescéine est dissous dans un triglycéride.

**7.** Utilisation d'un diester d'acide gras de fluorescéine et d'un saccharide choisi parmi le mannitol, le lactulose, le xylose, le rhamnose et le fructose pour fabriquer un agent diagnostique pour détecter l'insuffisance pancréatique.

Volume (ml)

600
500
400
300
200
100
0

0-2    2-4    4-6    6-8    8-10
Time(hrs)

▨ CFW=CF with enzymes

▨ CFWO=CF without enzymes

▨ NORMAL

COMBINED FDL-M TEST:
NORMAL v CF URINARY VOLUMES

Fluorescein (% total dose administered)

10
8
6
4
2
0

0-2    2-4    4-6    6-8    8-10
Time (hrs)

▨ CFW=CF with enzymes

▨ CFWO=CF without enzymes

▨ NORMAL

COMBINED FDL-M TEST:
NORMAL v CF FLUORESCEIN RECOVERY

8

COMBINED FDL-M TEST:
NORMAL v CF MANNITOL RECOVERY

COMBINED FDL-M TEST:
NORMAL v CF PERCENTAGE RATIOS